# NOUVEAU FASCICULE DE BREVET EUROPEEN

(11) **EP 0 603 176 B2**
(45) Date de publication et mention de la décision concernant l'opposition: **18.06.2003**
(45) Mention de la délivrance du brevet: 20.08.1997
(21) Numéro de dépôt: 92904034.3
(22) Date de dépôt: 16.01.1992
(51) Int. Cl.: C07C 229/34, C07D 305/14

(54) **LA beta-PHENYLISOSERINE-(2R,3S), SES SELS, SA PREPARATION ET SON EMPLOI**
BETA-PHENYLISOSERIN-(2R,3S), IHRE SALZE, HERSTELLUNG UND VERWENDUNG
beta-PHENYLISOSERINE-(2R,3S), SALTS, PREPARATION AND USE THEREOF

(30) Priorité: 17.01.1991 FR 9100491
(43) Date de publication de la demande: 29.06.1994
(73) Titulaire: Aventis Pharma S.A., 92160 Antony (FR)
(72) Inventeur: DUCHESNE, Jean-Pierre, F-69007 Lyon (FR); FERRARO, Max, F-69320 Feyzin (FR)
(74) Mandataire: Zumstein, Fritz, Dr.
(86) Numéro de dépôt international: FR9200032
(87) Numéro de publication internationale: WO92012958

(56) Documents cités:
- EP-A- 0 336 840
- EP-A- 0 336 841
- EP-A- 0 414 610
- ARCHIV DER PHARMAZIE, vol. 308, no. 2, février 1975, Weinheim, DE, pages 135-141; E.KAMANDI et al: "Die Synthese von Beta-Phenyl Isoserinen durch Ammonolyse von Beta-Phenylglycidestern. II."
- ARCHIV DER PHARMAZIE, vol. 307, no. 11, novembre 1974, Weinheim, DE, pages 871-878; E. KAMANDI et al: "Die Synthese von Beta-Phenyl Isoserinen durch Ammonolyse von Beta-Phenyl-Glycidestern. I"
- BULLETIN OF THE CHEMICAL SOCIETY OF JAPAN, vol. 47, novembre 1974, Tokyo, JP, pp. 2911-2912; H. HARADA et al: "Opticl resolution and configuration of cis-beta-phenylglycidic acid"
- JOURNAL OF ORGANIC CHEMISTRY, vol. 55, no. 6, 16 mars 1990, Washington, US, pages 1957-1959; J.-N. DENIS et al: "An improved synthesis of the taxol side chain and of RP56976"
- CHEMICAL ABSTRACTS, no. 115, 28 octobre 1991, Columbus, Ohio, US; abstract no. 183831G, page 985
- JOURNAL OF ORGANIC CHEMISTRY, vol. 56, no. 5, 1 mars 1991, Washington, US, pages 1681-1683; I. OJIMA et al: "Efficient and practical asymmetric synthesis of the taxol C-13 side chain, N-benzoyl-(2R,3S)-3-phenylisoserine, and its analogues via chiral ester enolate-imine cyclocondensation"
- J.Org.Chem. 1992, 4320-23
- TETRAHEDRON, 1990, 46, 3841f.
- J. AM. SOC., 1966, 100, 5917f.
- J. ORG. CHEM., 1958, 23, 742f.
- J. ORG. CHEM., 1986, 51, 46f.
- HOUBEN-WEYL, Band E5, 4. Auflage, Georg Thieme Verlag, 1985, S. 223ff
- ORGANIKUM, VEB Verlag, Berlin, 1986, S. 407; Lehrbuch der organischen Chemie, Bayer-Walter, Hirzel-Verlag, Stuttgart, 1991, S. 549
- GRUNDLAGEN DER ORG. CHEMIE, H. R. Christen et al, Otto Salle Verlag GmbH & Co., 1989. S. 175 ff.
- REAKTIONSKINETIK I, K. J. Laidler, Bibleographisches Institut Mannheim/Wien/Zürich, 1970, 43
- LEHRBUCH DER ORG. CHEMIE, Bayer-Walter, Hizel-Verlag, Stuttgart, 1991, S. 2; Organikum, VEB Verlag, Berlin, 1986, S. 133
- HANDBOOK OF CHEMISTRY AND PHYSICS, 66th ed., CRC Press, D-159, D-163

## Description

La présente invention concerne les sels alcalins ou alcalino-terreux ou les sels avec les bases azotées la β-phénylisosérine-(2R,3S) de formule : 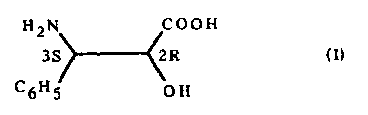 leur préparation et leur emploi pour la préparation de produits thérapeutiquement actifs.

Alors que les isomères (2R,3R), (2S,3S) et (2S,3R) de la β-phénylisosérine sont connus dans la littérature scientifique, par exemple dans les articles de E. Kamandi et coll., Arch. Pharmaz., 307, 871-878 (1974), de E. Kamandi et coll., Arch. Pharmaz., 308, 135-141 (1975) ou de K. Harada et Y Nakajima, Bull. Chem. Soc. Japan, 47, 2911-2912 (1974), la β-phénylisosérine-(2R,3S) ne paraît pas encore avoir été décrite autrement que sous forme d'ester [H. Hönig et coll., Tetrahedron, 46 (11) 3841-3850 (1990)].

Selon l'invention la β-phénylisosérine-(2R,3S), éventuellement sous forme de sel, est obtenue par action d'une solution aqueuse d'ammoniaque sur le sel d'ammonium de l'acide β-phénylglycidique-(2R,3R) à raison de 10 à 100 moles d'ammoniac et de préférence de 50 à 80 moles par mole d'acide β-phénylglycidique-(2R,3R) à une température comprise entre 0 et 100°C, de préférence entre 40 et 60°C. Le sel d'ammonium de l'acide β-phénylglycidique-(2R,3R) peut être obtenu par déplacement d'un sel de l'acide β-phinylglycidique-(2R,3R) choisi parmi les sels alcalins ou alcalino-terreux et les sels avec les bases azotées.

L'ammoniac est de préférence utilisé sous forme d'une solution aqueuse concentrée telle qu'une solution dont la concentration est comprise entre 20 et 32 % (p/p) à une température voisine de 25°C.

Généralement, on opère sous pression atmosphérique ou bien sous une pression autogène qui est voisine de 2,5 bars à 60°C.

Afin d'accélérer la réaction, il est particulièrement avantageux d'opérer en présence d'un sel d'ammonium tel que le chlorure d'ammonium ou d'hydrogénocarbonate d'ammonium. Il est préférable d'utiliser l'hydrogénocarbonate d'ammonium qui permet d'augmenter la vitesse de réaction tout en conservant la sélectivité. Généralement, on utilise une quantité stoechiométrique de sel d'ammonium par rapport à l'acide β-phénylglycidique mis en oeuvre.

Généralement, le procédé est mis en oeuvre en utilisant le sel de l'acide β-phénylglycidique-(2R,3R) avec l'α-méthylbenzylamine.

Cependant, on peut aussi utiliser un sel de métal alcalin (sodium, potassium) qui est obtenu par action d'une base (soude, potasse) en quantité stoechiométrique sur le sel de l'acide β-phénylglycidique-(2R,3R) avec l'α-méthylbenzylamine, ou le sel d'ammonium qui est obtenu par déplacement du sel de l'acide β-phénylglycidique-(2R,3R) avec l'α-méthylbenzylamine par l'ammoniaque en excès. Dans ce dernier cas, il est possible de favoriser le déplacement par extraction continue ou semi-continue de l'α-méthylbenzylamine au moyen d'un solvant organique convenable tel que le toluène.

Il est particulièrement intéressant d'utiliser le sel d'ammonium de l'acide β-phénylglycidique-(2R,3R) qui permet de rendre l'ouverture au moyen de l'ammoniac à la fois régiosélective et stéréosélective.

Quelle que soit la manière dont l'action de l'ammoniac sur le sel d'ammonium de l'acide β-phénylglycidique-(2R,3R) est mise en oeuvre, la β-phénylisosérine-(2R,3S) peut être isolée selon l'une des méthodes suivantes :
1) l'excès d'ammoniac peut être éliminé sous pression réduite de façon à obtenir le sel d'ammonium de l'acide β-phénylglycidique-(2R,3S) en solution aqueuse. Après addition d'un acide minéral fort, la β-phénylisosérine-(2R, 3S) précipite et est séparée par filtration, ou bien
2) avant, pendant ou après l'élimination de l'ammoniac sous pression réduite, il est possible d'ajouter une base alcaline (soude, potasse) ou alcalino-terreuse (chaux vive ou éteinte) ; le sel formé précipite après addition éventuelle d'un solvant organique tel que l'acétone. Le sel alcalin ou alcalino-terreux ainsi obtenu est séparé par filtration. Afin de faciliter le relargage du sel, en particulier du sel de sodium, de la β-phénylisosérine-(2R,3S), et d'améliorer le rendement, il peut être avantageux de saturer l'eau présente dans le mélange réactionnel par addition de chlorure de sodium.

L'acide β-phénylglycidique-(2R,3R) peut être préparé dans les conditions décrites par J-N. Denis et al., J. Org. Chem., 51, 46-50 (1986).

La β-phénylisosérine-(2R,3S) obtenue par la mise en oeuvre du procédé selon l'invention est particulièrement utile pour réaliser la synthèse de produits thérapeutiquement actifs tels que les dérivés du taxane de formule générale : 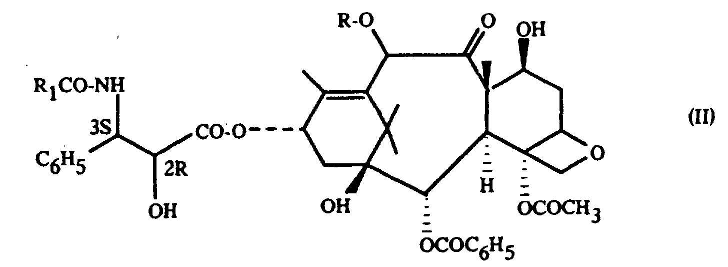 dans laquelle R représente un atome d'hydrogène ou un radical acétyle et R₁ représente un radical phényle ou t.butoxy.

Par action d'un agent de benzoylation (chlorure de benzoyle) ou de t.butoxycarbonylation (dicarbonate de t.butyle) puis d'un agent de protection de la fonction hydroxy, la β-phénylisosérine conduit au produit de formule générale : 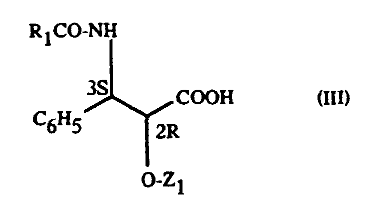 dans laquelle R₁ représente un radical phényle ou t.butoxy et Z₁ représente un groupement de la fonction hydroxy (éthoxy-1 éthyle).

Par condensation de l'acide de formule générale (III) sur la baccatine III ou la désacétyl-10 baccatine III dont les fonctions hydroxy en position -7 et éventuellement en position -10 sont protégées par des groupements protecteurs (radicaux silylés, trichloro-2,2,2 éthoxycarbonyle), suivie du remplacement des groupements protecteurs par des atomes d'hydrogène, est obtenu le produit de formule générale (II).

La condensation de l'acide de formule générale (III) sur la baccatine III ou la désacétyl-10 baccatine III protégées ainsi que le remplacement des groupements protecteurs par des atomes d'hydrogène peut être réalisée dans les conditions décrites dans le brevet européen EP-0 336 840 OU EP-0 336 841.

L'exemple suivant, donné à titre non limitatif, montre comment l'invention peut être mise en pratique.

### EXEMPLE 1

Dans une colonne, on introduit 3 kg de β-phénylglycidate-(2R,3R) d'α-méthylbenzylamine, dont le titre est de 98 % et dont l'excès énantiomérique est supérieur à 98,5 %, et 15 litres d'ammoniaque à 32 % (p/p). En bas de colonne, on introduit, au moyen d'une pompe doseuse, du toluène au débit de 3 à 5 litres/heure. La solution toluénique qui décante en tête de colonne est évacuée par débordement. Après avoir introduit 18 litres de toluène, on ne détecte plus d'α-méthylbenzylamine dans l'extrait toluénique.

Dans un autoclave de 150 litres, on introduit la solution de β-phénylglycidate d'ammonium obtenue précédemment et 30 litres d'ammoniaque à 32 % (p/p). L'autoclave est fermé puis chauffé, en 1 heure, à 60°C, sous agitation. La pression est voisine de 2,5 bars. On poursuit l'agitation à 60°C pendant 5 heures puis on laisse refroidir à 18°C. L'ammoniac est éliminé par distillation sous pression réduite (100-700 mm de mercure; 13,3-93 kPa) à 24°C, après avoir ajouté 9 kg de chlorure de sodium et 0,42 kg de soude en pastilles dans 2,5 litres d'eau. Quand la pression dans l'appareil atteint 45 mm de mercure (6 kPa), le mélange réactionnel est chauffé à 48°C afin de dissoudre les sels puis est refroidi à une température comprise entre -5 et -8°C pendant 3 heures.

Les cristaux blancs obtenus sont séparés par filtration puis séchés à 40°C sous pression réduite (1 mm de mercure; 0,13 kPa). On obtient ainsi 1932 g de sel de sodium de la β-phénylisosérine-(2R,3S) fondant à 218°C.

Le spectre de résonance magnétique nucléaire du ¹³C du sel de sodium de la β-phénylisosérine-(2R,35) déterminé dans l'eau deutérée à 90 MHz est caractérisé par les déplacements chimiques (δ) suivants : 60,2 (¹JCH = 140 Hz) ; 80,0 (¹JCH = 147 Hz ; ²J = 2,6 Hz); 129,6; 130,2; 131,5; 144,4 et 181,6 ppm.

### EXEMPLE 2

Dans un réacteur de 250 litres, on introduit 10 kg de β-phénylglycidate-(2R,3R) d'α-méthylbenzylamine (35,08 moles), 15 litres d'eau et 20 litres de toluène puis on ajoute en 10 minutes 10 litres de soude 4N à une température voisine de 20°C. On agite pendant 1 heure. La phase aqueuse est séparée par décantation. La phase toluénique, qui contient l'α-méthylbenzylamine est conservée. La phase aqueuse est lavée par 2 fois 10 litres de toluène pour éliminer la totalité de α-méthylbenzylamine. Des phases toluéniques réunies peut être isolée l'a-méthylbenzylamine.

A la phase aqueuse, placée dans un réacteur de 250 litres, on ajoute 1,860 kg de chlorure d'ammonium et 162 litres d'ammoniaque à 20 % (p/v). On chauffe à 50°C puis maintient sous agitation pendant 17 heures. Après refroidissement à 35°C on ajoute 35 kg de chlorure de sodium puis maintient à cette température pendant 30 minutes. On laisse refroidir lentement (2 heures) à une température comprise entre 0 et 5°C puis maintient pendant 1 heure à cette température. Le précipité est séparé par filtration puis séché sous pression réduite à 50°C. On obtient ainsi 7 kg de produit sec qui contient environ 25 % de chlorure de sodium et environ 5,300 kg de sel de sodium de la β-phénylisosérine-(2R,3S) pur.

Le rendement est de 72 %.

Le produit ainsi obtenu peut être utilisé tel quel dans les opérations de synthèse ultérieures.

## Revendications

1. Procédé de préparation de la β-phénylisosérine-(2R,3S), éventuellement sous forme de sel pour lequel on fait agir une solution aqueuse d'ammoniac sur un sel l'acide β-phénylglycidique-(2R,3R), à raison de 10 à 100 moles d'ammoniaque par mole d'acide β-phénylglycidique-(2R,3R) à une température comprise entre 0 et 100°C, **caractérisé en ce qu'**on utilise
le sel d'ammonium de l'acide β-phénylglycidique-(2R,3R),
et isole la β-phénylisosérine-(2R,3S) éventuellement sous forme de sel.

2. Procédé selon la revendication précédente **caractérisé en ce que** le sel d'ammonium de l'acide β-phénylglycidique-(2R,3R) est obtenu par déplacement d'un sel de l'acide β-phénylglycidique-(2R,3R) choisi parmi les sels alcalins ou alcalino-terreux et les sels avec les bases azotées.

3. Procédé selon la revendication 2 **caractérisé en ce que** le déplacement est effectué à partir d'un sel alcalin ou alcalino-terreux de l'acide β-phénylglycidique-(2R,3R) par action d'un sel d'ammonium choisi parmi le chlorure d'ammonium et l'hydrogénocarbonate d'ammonium

4. Procédé selon l'une quelconque des revendications précédentes **caractérisé en ce que** la base azotée est l'α-méthylbenzylamine.

5. Procédé selon la revendication 4 **caractérisé en ce que** le déplacement est effectué à partir d'un sel de l'acide β-phénylglycidique-(2R,3R) avec les bases azotées par action de l'ammoniac en excès et par extraction continue ou semi-continue de la base azotée au moyen d'un solvant organique.

6. Procédé de préparation de dérivés du taxane de formule générale : 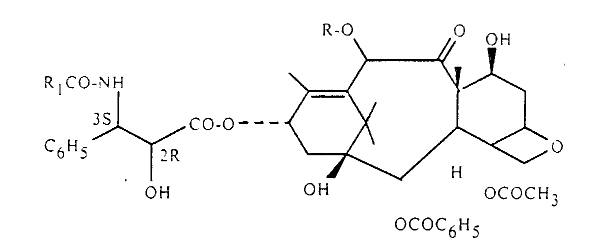 dans laquelle R représente un atome d'hydrogène ou un radical acétyle et R₁ représente un radical phényle ou t.butoxy, pour lequel on procède à partir de :
■ la β-phénylisosérine-(2R,3S) par action d'un agent de benzoylation ou de t.butoxycarbonylation, puis
■ un agent de protection de la fonction hydroxy sur la β-phénylisosérine-(2R,3S), puis
■ condense le produit obtenu sur la baccatine III ou la désacétyl-10 baccatine III dont les fonctions hydroxy en -7 et éventuellement en -10 sont protégées, puis,
■ après remplacement des groupements protecteurs des fonctions hydroxy par des atomes d'hydrogène, isole le produit obtenu,
**caractérisé en ce que** :
la β-phénylisosérine-(2R,3S) est obtenue, selon le procédé de la revendication 1.

7. Les sels de la β-phénylisosérine-(2R,3S) avec les métaux alcalins ou alcalino-terreux ou les bases azotées.

## Patentansprüche

1. Verfahren zur Herstellung von β-Phenylisoserin-(2R,3S), gegebenenfalls in Form von Salz, bei dem man eine wäßrige Lösung von Ammoniak mit einem Salz der β-Phenylglycidinsäure-(2R,3R) in einem Verhältnis von 10 bis 100 Mol Ammoniak pro Mol β-Phenylglycidinsäure-(2R,3R) bei einer Temperatur zwischen 0 °C und 100 °C zur Reaktion bringt, **dadurch gekennzeichnet, daß** man das Ammoniumsalz der β-Phenylglycidinsäure-(2R,3R) verwendet und das β-Phenylisoserin-(2R,3S) gegebenenfalls in Form von Salz isoliert.

2. Verfahren nach dem vorstehenden Anspruch, **dadurch gekennzeichnet, daß** das Ammoniumsalz der β-Phenylglycidinsäure-(2R,3R) durch Umlagerung eines Salzes der β-Phenylglycidinsäure-(2R,3R), ausgewählt unter den Alkalisalzen oder den Erdalkalisalzen und den Salzen mit Stickstoffbasen, erhalten wird.

3. Verfahren nach Anspruch 2, **dadurch gekennzeichnet, daß** die Umlagerung ausgehend von einem Alkalisalz oder Erdalkalisalz der β-Phenylglycidinsäure-(2R,3R) mittels Einwirkung eines Ammoniumsalzes, ausgewählt unter Ammoniumchlorid und Ammoniumhydrogencarbonat, durchgeführt wird.

4. Verfahren nach irgendeinem der vorstehenden Ansprüche, **dadurch gekennzeichnet, daß** die Stickstoffbase α-Methylbenzylamin ist.

5. Verfahren nach Anspruch 4, **dadurch gekennzeichnet, daß** die Umlagerung ausgehend von einem Salz der β-Phenylglycidinsäure-(2R,3R) mit der Stickstoffbase mittels Einwirkung von Ammoniak im Überschuß und mittels kontinuierlicher oder halbkontinuierlicher Extraktion der Stickstoffbase mit Hilfe eines organischen Lösungsmittels durchgeführt wird.

6. Verfahren zur Herstellung von Taxan-Derivaten der allgemeinen Formel 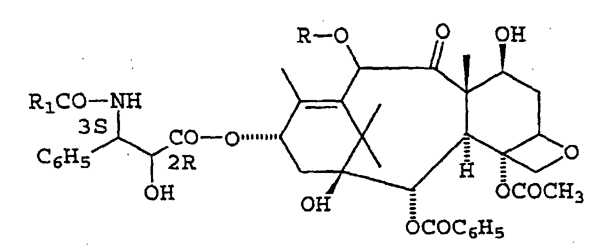 in der R ein Wasserstoffatom oder einen Acetylrest darstellt und R₁ einen Rest Phenyl oder tert.-Butoxy bedeutet, bei dem man verfährt ausgehend von:
■ β-Phenylisoserin-(2R,3S), durch Einwirkung eines Mittels zur Benzoylierung oder zur tert.-Butoxycarbonylierung, anschließend
■ eines Mittels zum Schutz der Hydroxyfunktion am β-Phenylisoserin-(2R,3S), danach
■ kondensiert man das erhaltene Produkt mit Baccatin III oder 10-Desacetyl-Baccatin III, deren Hydroxyfunktionen in -7 und eventuell in -10 geschützt sind, anschließend,
■ nach dem Austausch der Schutzgruppen an den Hydroxyfunktionen durch Wasserstoffatome, isoliert man das erhaltene Produkt,
**dadurch gekennzeichnet, daß** das β-Phenylisoserin-(2R,3S) gemäß dem Verfahren von Anspruch 1 erhalten wird.

7. Die Salze von β-Phenylisoserin-(2R,3S) mit Alkalimetallen oder Erdalkalimetallen oder Stickstoffbasen.

## Claims

1. Process for preparing (2R,3S)-β-phenylisoserine, optionally in salt form, for which process aqueous ammonia solution is reacted with a salt of (2R, 3R)-β-phenylglycidic acid, in the proportion of 10 to 100 mol of ammonia solution per mole of (2R,3R)-β-phenylglycidic acid at a temperature of between 0 and 100°C, **characterized in that**
the ammonium salt of (2R,3R)-β-phenylglycidic acid is used,
and the (2R,3S)-β-phenylisoserine is isolated, optionally in salt form.

2. Process according to the preceding claim, **characterized in that** the ammonium salt of (2R,3R)-β-phenylglycidic acid is obtained by displacement of a salt of (2R,3R)-β-phenylglycidic acid chosen from the alkali metal or alkaline earth metal salts and the salts with nitrogenous bases.

3. Process according to Claim 2, **characterized in that** the displacement is carried out starting from an alkali metal or alkaline earth metal salt of (2R,3R)-β-phenylglycidic acid by the action of an ammonium salt chosen from ammonium chloride and ammonium hydrogen carbonate.

4. Process according to any one of the preceding claims, **characterized in that** the nitrogenous base is α-methylbenzylamine.

5. Process according to Claim 4, **characterized in that** the displacement is carried out starting from a salt of (2R,3R)-β-phenylglycidic acid with the nitrogenous base by the action of an excess of ammonia, and by continuous or semi-continuous extraction of the nitrogenous base by means of an organic solvent.

6. Process for preparing taxane derivatives of general formula: 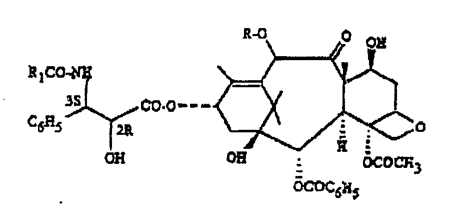 in which R represents a hydrogen atom or an acetyl radical and R₁ represents a phenyl or t-butoxy radical, which process starts from:
■ (2R,3S)-β-phenylisoserine by the action of a benzoylating or t-butoxycarbonylating agent, then
■ an agent for protection of the hydroxyl function on the (2R,3S)-β-phenylisoserine, then
■ the product obtained is condensed with baccatin III or 10-deacetylbaccatin III in which the hydroxyl functions at the 7- and, where appropriate, the 10-position(s) are protected, and then,
■ after replacement of the groups protecting the hydroxyl functions with hydrogen atoms, the product obtained is isolated,
**characterized in that**:
the (2R,3S)-β-phenylisoserine is obtained according to the process of Claim 1.

7. The salts of (2R,3S)-β-phenylisoserine with alkali metals or alkaline earth metals or nitrogenous bases.
